# EUROPÄISCHE PATENTSCHRIFT

(11) **EP 3 843 697 B1**
(45) Veröffentlichungstag und Bekanntmachung des Hinweises auf die Patenterteilung: **09.07.2025**
(21) Anmeldenummer: 19728395.5
(22) Anmeldetag: 31.05.2019
(51) Int. Cl.: A61K 8/44, A61K 8/46, A61Q 19/10

(54) **MILDE REINIGUNGSZUBEREITUNG II**
MILD CLEANING PREPARATION II
PRÉPARATION NETTOYANTE DOUCE II

(30) Priorität: 29.08.2018 DE 102018214573
(43) Veröffentlichungstag der Anmeldung: 07.07.2021
(73) Patentinhaber: Beiersdorf AG, 22529 Hamburg (DE)
(72) Erfinder: NILSSON, Jan, 22177 Hamburg (DE)
(74) Vertreter: Beiersdorf AG
(86) Internationale Anmeldenummer: PCT/EP2019/064164
(87) Internationale Veröffentlichungsnummer: WO 2020/043336

(56) Entgegenhaltungen:
- US-A1- 2015 157 548
- DR TONY GOUGH: "Formulating High Performance, Sulfate- Free Cleansing Products Content", 16 April 2015 (2015-04-16), XP055607134, Retrieved from the Internet <URL:https://www.in-cosmetics.com/RXUK/RXUK_InCosmetics/2015-Website/Documents/in-cos15,IS,T3,D3,Formulating%20high%20performance,%20sulfate-free%20cleansing%20products,Dr.%20Tony%20Gough.pdf?v=635653985524043037> [retrieved on 20190719]
- DATABASE GNPD [online] MINTEL; 26 October 2015 (2015-10-26), ANONYMOUS: "Body Wash", XP055607240, retrieved from www.gnpd.com Database accession no. 3474535
- DATABASE GNPD [online] MINTEL; 7 January 2011 (2011-01-07), ANONYMOUS: "Hair & Body Wash", XP055607232, retrieved from www.gnpd.com Database accession no. 1459109
- DATABASE GNPD [online] MINTEL; 19 April 2018 (2018-04-19), ANONYMOUS: "Bubble Bath", XP055607236, retrieved from www.gnpd.com Database accession no. 5595855
- DATABASE GNPD [online] MINTEL; 8 March 2018 (2018-03-08), ANONYMOUS: "Purifying Body Wash", XP055607238, retrieved from www.gnpd.com Database accession no. 5506663

## Beschreibung

Kosmetische Produkte dienen im Allgemeinen nicht nur dazu schön und attraktiv auszusehen, sondern sie tragen mit ihrer Wirkung entscheidend zu einem gesteigerten Selbstwertgefühl und zum Wohlbefinden der Menschen bei. Dementsprechend werden die verschiedensten kosmetischen Produkte zur täglichen Reinigung und Pflege der menschlichen Haut eingesetzt.

Insbesondere Reinigungszubereitungen für Babys müssen erhöhte Anforderungen für eine sanfte und angenehme Reinigung erfüllen, so dass es bei Ihrer Anwendung zu keinen Hautirritationen und/oder einem brennen bei Augenkontakt kommt. Um diesen Anforderungen genüge zu tragen besteht ein ständiger Bedarf an Formulierungen, welche besonders zur Reinigung von Babyhaut geeignet sind.

Wie unter anderem die EP 1010422 A2 ausführt, ist es für Reinigungszubereitungen für Babys ebenfalls entscheidend, dass die Reinigungszubereitung ein Verdickersystem enthält, um die Möglichkeit zu minimieren, dass das Produkt in die Augen fließt. Betrachtet man jedoch die derzeit eingesetzten Verdickersysteme für Reinigungszubereitungen, so weisen diese insbesondere für den Einsatz auf sensibler Babyhaut erhebliche Nachteile auf, welche im Folgenden dargestellt werden.

Beispielsweise können Reinigungszubereitungen mit PEG-haltigen Tensidsystemen oftmals durch die Zugabe von Salzen oder Elektrolyten verdicket werden. Ein einsetzbares Tensid ist Natriumlaurylethersulfat, vorteilhaft in Kombination mit Betainen. Als Salz kann unter anderem Natriumchlorid eingesetzt werden. Jedoch sind solche Verdickersysteme für den Einsatz in hautfreundlichen Reinigungszubereitungen für Babys ungeeignet, da bekanntermaßen Natriumlaurylethersulfat ein erhöhtes Hautirritationspotential aufweist. Ferner stehen PEG-haltige Substanzen zunehmend in der Kritik, da diese im Verdacht stehen die Haut durchlässiger zu machen, indem sie die natürliche Schutzbarriere der Haut abschwächen. Insbesondere bei Babyprodukte sollte somit auf derartige Substanzen verzichtet werden.

Eine Salzverdickung/Elektrolytverdickung, wie vorstehend für die PEG-haltigen Tenside beschrieben, ist bei ausschließlicher Verwendung von hautverträglichen amphoteren oder nichtionischen Tensiden nicht oder nur sehr begrenzt möglich. Vielmehr führt die Zugabe von Salzen, wie Natriumchlorid, oftmals zu einem schnellen Viskositätsverlust.

Alternativ zu der Salz/Elektrolytverdickung ist es ein standartmäßiges Verfahren, milden Reinigungszubereitungen verdickende Acrylat-basierte Polymere oder weitere verdickende Substanzen zuzusetzen. Typische Verdicker sind beispielsweise PEG-haltige Verdicker, wie PEG-120 Methyl Glucose Dioleat oder PEG-200 Hydrogenated Glyceryl Palmate, welche ebenfalls, wie vorstehend für die PEG-haltigen Tenside beschrieben, in der Kritik stehen und somit nicht eingesetzt werden sollten. Zu den Acrylat-basierten Polymeren zählen Polymere, welche aus Homo- oder Copolymerisation mit Acryl- und/oder Methacrylsäure erhalten werden. Beispiele hierfür sind u.a. Carbomer oder Acrylate Copolymer. Jedoch ist auch der Einsatz dieser Acrylat-basierten Polymere zunehmend in der Kritik, da deren biologische Abbaubarkeit nicht vollständig geklärt ist.

Weiterhin ist es dem Fachmann bekannt die Polymere Xanthan Gum, Cellulosen und Cellulosederivate und/oder Stärken und Stärkederivate als Verdicker einzusetzen. Diese Biopolymere stehen zwar nicht in der Kritik, dass sie biologisch nicht abbaubar sind, weisen bei Einsatz zur Verdickung von Reinigungszubereitungen jedoch erhebliche Nachteile auf. So zeigen verdickende Biopolymere oftmals einen negativen Einfluss auf die mikrobiologische Stabilität, welche nur über einen zusätzlichen Einsatz von Konservierungsmitteln kompensiert werden kann. Ein weiterer negativer Aspekt dieser Biopolymere ist, dass diese oftmals schwankende Mengen an Elektrolyten enthalten. Das hat zur Folge, dass die einzelnen Reinigungszubereitungen nicht gleichmäßig zu produzieren sind und diese abhängig von der Elektrolytmenge in den Rohstoffen nach Produktion oftmals unterschiedliche rheologische Eigenschaften/Viskositäten aufweisen. Somit weisen die produzierten Reinigungszubereitungen aus unterschiedlichen Chargen oftmals unterschiedliche Eigenschaften auf, was bei Massenware zu verhindern ist, da der Konsument gleichbleibende Qualität erwartet.

Ein weiterer negativer Aspekt ist, dass die Polymere Xanthan Gum, Cellulosen und Cellulosederivate und/oder Stärken und Stärkederivate sowie Acrylat-basierte Polymere das Schaumverhalten von Reinigungszubereitungen negativ beeinflussen. So wird bei deren Einsatz das erhaltene Schaumvolumen der Reinigungszubereitung reduziert. Eine Kompensation erfolgt oft mit hohen Konzentrationen an PEG-haltigen Tensiden, wie Natriumlaurylethersulfat, was wiederum zu einem erhöhten Hautirritationspotential führt.

Generell ist es entscheidend, dass die Reinigungszubereitungen eine Viskosität im Bereich von 1000 bis 4000 mPa·s, bevorzugt 1500 bis 3100 mPa·s aufweisen. So wird sichergestellt, dass diese sich auf der Haut verteilen lassen, ohne dass diese abrutschen. Gleichzeitig ist eine einfache Entnahme aus Tuben oder Quetschflaschen möglich.

Ein weiterer Nachteil von bekannten Reinigungszubereitungen ist vor allem der Umstand, dass die Viskosität dieser Reinigungszubereitungen sich oftmals bei Lagerung im Packmittel reduziert, so dass die Produkte nach mehreren Wochen Lagerung nicht mehr den gewünschten Viskositätsbereich aufweisen. Somit wird eine Viskosität im Bereich von 1000 bis 4000 mPa·s, bevorzugt 1500 bis 3100 mPa·s nicht mehr erzielt und die Reinigungszubereitung ist meist zu dünnflüssig. Diese Problematik kann im Labor durch Lagerung unter Normalbedingen für 240 Tage nach Herstellung untersucht werden.

Dementsprechend besteht weiterhin ein Bedarf an Mitteln zur Bereitstellung verdickter Reinigungszubereitungen, insbesondere für Babys. Insbesondere war es Aufgabe der vorliegenden Erfindung Reinigungszubereitungen bzw. Verdickersysteme für Reinigungszubereitungen Bereitzustellen, welche die Nachteile des Standes der Technik nicht aufweisen. Insbesondere sollten die Reinigungszubereitungen nach der Lagerung über einen Zeitraum von 240 Tagen nach Herstellung eine Viskosität im Bereich von 1000 bis 4000 mPa·s, bevorzugt 1500 bis 3100 mPa·s aufweisen.

Das Dokument "Formulating High Performance, Sulfate- Free Cleansing Products Content", 16 April 2015 von Dr. Tony Gouqh beschreibt die verdickende Wirkung von Tensiden. Weiterhin kennt der Fachmann das Dokument US 2015/157548 A1 welches sulfatfreie kosmetische Reinigungszubereitung beschreibt. Jedoch konnte keines dieser Dokumente einen Hinweis auf die Erfindung geben.

Überraschend für den Fachmann wurde nun gefunden, dass der kombinierte Einsatz von spezifischen Tenside zu einer synergistischen Verdickung führt. Weiterhin zeigte diese spezifische Kombination die aufgeführten Nachteile der vorstehend beschriebenen Verdickersysteme überraschend nicht aufweist.

Gegenstand der vorliegenden Erfindung ist daher eine kosmetische Reinigungszubereitung enthaltend, bezogen auf das Gesamtgewicht der Reinigungszubereitung,
a) Sodium Lauroyl Methyl Isethionate,
b) Sodium Methyl Cocoyl Taurate, und
c) Cocamidopropylbetaine,
dadurch gekennzeichnet, dass Cocamidopropylbetaine in einem Anteil von 3,5 Gew.-% bis 6 Gew.-% enthalten ist und Wasser in einem Anteil von 60 Gew.-% bis 94 Gew.-% enthalten ist.

Wie die vorliegenden Vergleichsversuche zeigen, zeigt die erfindungsgemäße Kombination eine synergistische Verdickung der Reinigungszubereitung.

Sollten nachfolgend Gewichtsprozentangaben (Gew.-%) ohne Bezugnahme auf eine bestimmte Zusammensetzung oder spezifische Mischung angegeben werden, so beziehen sich diese Angaben immer auf das Gesamtgewicht der kosmetischen Reinigungszubereitung. Sollten nachfolgend Verhältnisse von Komponenten/Substanzen/Stoffgruppen offenbart werden, so beziehen sich diese Verhältnisse auf Gewichtsverhältnisse der genannten Komponenten/Substanzen/Stoffgruppen.

Werden nachfolgend Gewichtsprozentbereiche für die Bestandteile der kosmetischen Reinigungszubereitung angegeben, so umfasst die Offenbarung der vorliegenden Anmeldung ebenfalls alle Einzelwerte in Schritten von 0,1 Gew.-% innerhalb dieser Gewichtsprozentbereiche.

Die Formulierungen "erfindungsgemäß", "erfindungsgemäß vorteilhaft", "vorteilhaft im Sinne der Vorliegenden Erfindung" etc. beziehen sich im Rahmen der vorliegenden Offenbarung immer sowohl auf die erfindungsgemäße Zubereitung sowie die erfindungsgemäße Verwendung und das erfindungsgemäße Verfahren.

Werden in dieser Offenbarung Viskositätswerte angegeben, so beziehen sich alle Werte auf eine Messung bei 25°C im 150 ml Rollrandglas mittels Rheomat R 123 von der Firma proRheo. Der Rheomat R 123 der Firma proRheo GmbH ist ein Rotationsviskosimeter, d. h. ein Messkörper rotiert in der zu vermessenden Substanz. Es wird die Kraft gemessen, die benötigt wird, um den Messkörper in der Probe mit einer vorgegebenen Drehzahl rotieren zu lassen. Aus diesem Drehmoment, der Drehzahl des Messkörpers und den geometrischen Abmessungen des verwendeten Messsystems wird die Viskosität berechnet. Als Messkörper wird der Messkörper Nr.1 (Artikelnr. 200 0191), geeignet für einen Viskositätsbereich bis 10.000 [mPa·s], Drehzahl Bereich 62,5 min⁻¹, verwendet. Erfolgt keine andere Angabe, so erfolgt die Messung zur Viskosität immer 24h nach Herstellung der Reinigungszubereitung. Das gleiche gilt für alle angegeben Viskositätswerte, welche sich immer auf eine Messung 24h nach Herstellung beziehen, sofern keine andere Zeitangabe angegeben ist.

Sofern nicht anders angegeben wurden alle Versuche unter Normalbedingungen durchgeführt. Der Begriff "Normalbedingungen" bedeutet 20°C, 1013 hPa und eine relative Luftfeuchtigkeit von 50%.

Erfindungsgemäß vorteilhaft beträgt der Anteil an Sodium Lauroyl Methyl Isethionate in der kosmetischen Reinigungszubereitung von 0,5 Gew.-% bis 5 Gew.-%, jeweils bezogen auf das Gesamtgewicht der Reinigungszubereitung.

Kommerziell kann Sodium Lauroyl Methyl Isethionate unter dem Handelsnamen Isulex^{®} LQ-CLR-SB von Innospec Performance Chemicals bezogen werden.

Weiterhin ist es erfindungsgemäß vorteilhaft, wenn der Anteil von Sodium Methyl Cocoyl Taurate von 0,5 Gew.-% bis 4 Gew.-%, jeweils bezogen auf das Gesamtgewicht der Reinigungszubereitung, beträgt.

Sodium Methyl Cocoyl Taurate kann ebenfalls von der Firma Innospec Performance Chemicals bezogen werden.

Weiterhin ist die erfindungsgemäße kosmetische Reinigungszubereitung vorteilhaft dadurch gekennzeichnet, dass diese einen pH-Wert im Bereich von 4,4 bis 6,5 und bevorzugt von 5 bis 5,5 aufweist.

Darüber hinaus ist die erfindungsgemäße kosmetische Reinigungszubereitung bevorzugt dadurch gekennzeichnet, dass diese keine mit Polyethylen Glykol substituierte Substanzen enthält. Somit sind bevorzugt keine PEG-haltigen Substanzen enthalten.

Es ist zwar generell erfindungsgemäß möglich zur weiteren Verdickung der kosmetischen Reinigungszubereitung Acrylat-basierte Polymere zuzusetzen, jedoch ist es bevorzugt auf den Einsatz von Acrylat-basierten Polymeren zu verzichteten, da die gewünschte Verdickung der Zubereitung durch die spezifische Kombination der Tenside erreicht wird. Daher ist die erfindungsgemäße kosmetische Reinigungszubereitung bevorzugt dadurch gekennzeichnet, dass die diese keine Acrylat-basierten Polymere enthält.

Es ist ebenfalls erfindungsgemäß möglich zur weiteren Verdickung weitere Polymere auf Basis von Polysacchariden und/oder Stärken der kosmetischen Reinigungszubereitung zuzusetzen, jedoch ist es bevorzugt auf den Einsatz von weitere Polymere auf Basis von Polysacchariden und/oder Stärken zu verzichteten, da die gewünschte Verdickung der Zubereitung durch die spezifische Kombination der Tenside erreicht wird. Daher ist die erfindungsgemäße kosmetische Reinigungszubereitung bevorzugt dadurch gekennzeichnet, dass die diese keine zusätzlichen Polymere auf Basis von Polysacchariden und/oder Stärken enthält.

Weiterhin ist es vorteilhaft im Sinne der vorliegenden Erfindung, wenn die kosmetische Reinigungszubereitung kein Natriumlaurylethersulfat oder Natriumaurylsulfat enthält.

Eine erste vorteilhafte Ausführungsform der vorliegenden Erfindung ist ferner dadurch gekennzeichnet, dass neben den erfindungsgemäßen Tensiden keine weiteren Tenside in der erfindungsgemäßen Reinigungszubereitung enthalten sind. So wurde bei Einsatz der erfindungsgemäßen Tenside bereits eine überraschend gute Reinigungsleistung erzielt. Überraschend war diese erfindungsgemäße Kombination an Tensiden ebenfalls besonders milde zur Haut, so dass die Reinigungszubereitung besonders zur Reinigung von Babyhaut, also bis zu einem Lebensalter von 3 Jahren, geeignet ist.

Wie für die erste Ausführungsform der Erfindung bereits beschrieben, zeigt die erfindungsgemäße Reinigungszubereitung nicht nur eine effektive Reinigungsleistung, sondern ist auch noch besonders schonend für die Haut. Besteht jedoch ein Bedarf, das Reinigungsvermögen der Reinigungszubereitung weiter zu steigern, oder die Menge an gebildetem Schaum bezogen auf das Volumen des Schaums zu erhöhen, so ist es erfindungsgemäß in einer zweiten vorteilhaften Ausführungsform möglich weitere Tenside in der erfindungsgemäßen Reinigungszubereitung einzusetzen. Eine zweite vorteilhafte Ausführungsform der Erfindung ist somit dadurch gekennzeichnet, dass neben den erfindungsgemäßen Tensiden zusätzlich weitere Tenside enthalten sind.

Innerhalb der zweiten vorteilhaften Ausführungsform ist es bevorzugt, wenn als weiteres Tensid Sodium Cocoamphoacetate enthalten ist. Ist Sodium Cocoamphoacetate enthalten, so beträgt der Anteil von Sodium Cocoamphoacetate vorteilhaft von 0,1 Gew.-% bis 1 Gew.- % bezogen auf das Gesamtgewicht der Reinigungszubereitung.

Innerhalb der zweiten vorteilhaften Ausführungsform ist es auch bevorzugt, wenn als weiteres Tensid ein Alkylglucosid enthalten ist. Ist ein Alkylglucosid enthalten, so beträgt der Gesamtanteil der Alkylglucoside vorteilhaft von 0,1 Gew.-% bis 1,5 Gew.-% bezogen auf das Gesamtgewicht der Reinigungszubereitung. Alkylglucoside sind unter anderem unter den INCI Namen Decyl Glucoside, Lauryl Glucoside oder Coco-Glucoside bekannt.

Trotz dass weitere Tenside, wie vorteilhafter weise ein Alkylglucosid und/oder Sodium Cocoamphoacetate, in der zweiten vorteilhaften Ausführungsform der Erfindung enthalten sein dürfen, hat es sich weiterhin gezeigt, dass es dennoch vorteilhaft ist, wenn die Reinigungszubereitung dieser Ausführungsform kein Sodium Cocoylglutamate und/oder kein Sodium Lauroamphoacetate enthält.

Die nachfolgend beschriebenen Merkmale der erfindungsgemäßen Reinigungszubereitung beziehen sich auf die erfindungsgemäße Tensidkombination an sich, sowie jeweils auf die zuvor beschriebenen erfindungsgemäßen vorteilhaften Ausführungsformen.

Die erfindungsgemäßen kosmetischen Reinigungszubereitungen können weiterhin vorteilhaft Natriumbenzoat enthalten. Enthält die kosmetische Reinigungszubereitung Natriumbenzoat, so ist es erfindungsgemäß vorteilhaft, wenn der Anteil an Natirumbenzoat von 0,1 Gew.-% bis 0,5 Gew.-% bezogen auf das Gesamtgewicht der Reinigungszubereitung beträgt.

Weiterhin ist es vorteilhaft, wenn die kosmetische Reinigungszubereitung dadurch gekennzeichnet ist, dass diese Natriumsalicylat enthält und der Anteil von Natriumsalicylat bevorzugt von 0,1 Gew.-% bis 0,5 Gew.-% bezogen auf das Gesamtgewicht der Reinigungszubereitung beträgt.

Weiterhin sind vorteilhafte kosmetische Reinigungszubereitungen der Erfindung dadurch gekennzeichnet, dass diese ein oder mehrere Alkandiole mit 6 oder 8 Kohlenstoffatomen enthalten, wobei der Gesamtanteil diese Alkandiole mit 6 oder 8 Kohlenstoffatomen bevorzugt von 0,01 Gew.-% bis 0,4 Gew.-% bezogen auf das Gesamtgewicht der Reinigungszubereitung beträgt.

Zusätzlich ist es vorteilhaft im Sinne der vorliegenden Erfindung, wenn die kosmetische Reinigungszubereitung Glycerin enthält und der Anteil von Glycerin bevorzugt von 0,01 Gew.-% bis 10 Gew.-% bezogen auf das Gesamtgewicht der Reinigungszubereitung beträgt.

Weiterhin ist es vorteilhaft, wenn Natriumchlorid in einem Anteil von weniger als 0,1 Gew.-% bezogen auf das Gesamtgewicht der Reinigungszubereitung der erfindungsgemäßen Reinigungszubereitung zugesetzt wird, wobei es insbesondere bevorzugt ist, wenn der erfindungsgemäße Reinigungszubereitung kein Natriumchlorid zugesetzt wird.

Ferner ist es vorteilhaft, wenn die kosmetische Reinigungszubereitung der vorliegenden Erfindung dadurch gekennzeichnet ist, dass, sofern diese eine Ölphase aufweist, der Gesamtanteil der Ölphase bezogen auf das Gesamtgewicht der Reinigungszubereitung weniger als 1 Gew.-%, bevorzugt weniger als 0,8 Gew.-%, weiterhin bevorzugt weniger als 0,7 Gew.-% und insbesondere bevorzugt weniger als 0,6 Gew.-% beträgt. Erfindungsgemäß zählen zur Ölphase Fette, Wachse, Öle, Fettsäuren und Fettalkohole sowie flüssige Riechstoffe. Andere Tenside und Emulgatoren zählen definitionsgemäß nicht zur Ölphase.

Unter Emulgatoren werden alle Substanzen verstanden, welche im International Cosmetic Ingredient Dictionary and Handbook, Thirteenth Edition 2010, (ISBN 1-882621-47-6) unter der Bezeichnung "emulsifying agent" geführt werden. Unter Tensiden werden alle Substanzen verstanden, welche im International Cosmetic Ingredient Dictionary and Handbook, Thirteenth Edition 2010, (ISBN 1-882621-47-6) unter der Bezeichnung " surfactant " geführt werden.

Weitere vorteilhafte Ausführungsformen der Erfindung sind dadurch gekennzeichnet, dass diese zusätzlich Polyquaternium-7 und/oder Polyquaternium-10 enthalten und der Anteil von Polyquaternium-7 und/oder Polyquaternium-10 von 0,05 Gew.-% bis 0,3 Gew.-% bezogen auf das Gesamtgewicht der kosmetischen Reinigungszubereitung beträgt.

Weitere vorteilhafte Ausführungsformen der Erfindung sind dadurch gekennzeichnet, dass diese zusätzlich Hydroxypropyl Guar Hydroxypropyltrimonium Chloride und/oder Guar Hydroxypropyltrimonium Chloride enthalten und der Anteil von Hydroxypropyl Guar Hydroxypropyltrimonium Chloride und/oder Guar Hydroxypropyltrimonium Chloride von 0,05 Gew.-% bis 0,3 Gew.-% bezogen auf das Gesamtgewicht der kosmetischen Reinigungszubereitung beträgt.

Weiterhin ist es ebenfalls bevorzugt, wenn die kosmetischen Reinigungszubereitungen Wasser in einem Anteil von 60 Gew.-% bis 94 Gew.-%, weiterhin bevorzugt von 75 Gew.- % bis 94 Gew.-% und insbesondere bevorzugt von 85 Gew.-% bis 94 Gew.-% bezogen auf das Gesamtgewicht der kosmetischen Reinigungszubereitung enthalten.

Weiterhin hat sich überraschend für den Fachmann gezeigt, dass die Transparenz der kosmetischen Reinigungszubereitung erhöht werden kann, indem vor Mischen der Bestandteile Sodium Methyl Cocoyl Taurate separat aufgeschmolzen wird, und das flüssige Sodium Methyl Cocoyl Taurate der restlichen Reinigungszubereitung zugegeben wird, wobei die restliche Reinigungszubereitung vorteilhaft eine Temperatur im Bereich von 10°C bis 30°C Temperatur aufweist.

Vorteilhaft ist somit auch ein Verfahren zur Herstellung der erfindungsgemäßen kosmetischen Zubereitung dadurch gekennzeichnet, dass
a) Sodium Methyl Cocoyl Taurate wird aufgeschmolzen bis alle Feststoffe gelöst sind, und
b) Beide Tenside zum erhalten der erfindungsgemäßen kosmetischen Reinigungszubereitung zu den restlichen Bestandteilen der Reinigungszubereitung zugegeben werden, wobei die restlichen Bestandteile der Reinigungszubereitung vorteilhaft eine Temperatur im Bereich von 10°C bis 30°C aufweisen.

Die kosmetischen Reinigungszubereitungen gemäß der Erfindung können ferner kosmetische Hilfsstoffe und Wirkstoffe enthalten, wie sie üblicherweise in solchen Zubereitungen verwendet werden, z. B. weitere Wirkstoffe, Konservierungsmittel, Konservierungshelfer, Bakterizide, Lipide, Farbstoffe und Farbpigmente, Verdickungsmittel, anfeuchtende und/oder feuchthaltende Substanzen oder andere übliche Bestandteile einer kosmetischen oder dermatologischen Formulierung wie weitere Polyole, Schaumstabilisatoren, organische Lösungsmittel oder Silikonderivate, sofern der Zusatz die geforderten Eigenschaften hinsichtlich der Stabilität nicht beeinträchtigen oder ausgeschlossen sind.

### Vergleichsversuche und Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

In der nachfolgenden Tabelle werden die Beispielrezepturen Bsp.1 bis Bsp.4 aufgeführt, welche erfindungsgemäße Kombinationen offenbaren. Die Messung der Viskosität gemäß dem offenbarten Verfahren zeigt, dass es bei der erfindungsgemäßen Kombination bei längerer Lagerung zu keinen signifikanten Viskositätsänderungen kommt.

| **Inhaltsstoffe** | **Bsp.1** | **Bsp.2** | **Bsp.3** | **Bsp.4** |
|---|---|---|---|---|
| Sodium Lauroyl Methyl Isethionate | 1,27 | 1,61 | 1,61 | 1,61 |
| Sodium Methyl Cocoyl Taurate | 1,25 | 1,25 | 1,25 | 1,25 |
| Cocamidopropyl Betaine | 4,4 | 4.4 | 4,4 | 4,4 |
| Polyquaternium-7 | 0,2 | 0,2 | 0,2 | |
| Parfum | 0,5 | 0,4 | 0,5 | 0,5 |
| Citric Acid | | | 0,2 | |
| Sodium Benzoate | 0,45 | 0,45 | 0,45 | 0,45 |
| Sodium Chloride | | | | |
| Wasser | ad 100 | ad 100 | ad 100 | ad 100 |
| Gemessene Viskosität 24h nach Herstellung in mPa·s | 2600 | 2650 | 2900 | 2850 |
| Gemessene Viskosität nach 240 Tagen Lagerung unter Normalbedingungen | 2850 | 2800 | 3050 | 2700 |

Die Reinigungszubereitungen der vorliegenden Erfindung überraschend milde, so dass Sie sich besonders gut zur Anwendung auf der Babyhaut eignen.

| **Inhaltsstoffe** | **Bsp. 5** | **Bsp. 6** | **Bsp. 7** | **Bsp. 8** | **Bsp. 9** | **Bsp. 10** | **Bsp. 11** | **Bsp. 12** |
|---|---|---|---|---|---|---|---|---|
| Sodium Lauroyl Methyl Isethionate | 1,5 | 1,7 | 1,5 | 1,9 | 1,4 | 1,2 | 1,8 | 1,9 |
| Sodium Methyl Cocoyl Taurate | 1,25 | 1,4 | 1,3 | 1,1 | 1,5 | 1,6 | 1,15 | 1,45 |
| Cocamidopropyl Betaine | 4,4 | 4,1 | 4,8 | 4 | 5 | 4,2 | 4,5 | 3,5 |
| Polyquaternium-7 | 0,2 | 0,225 | 0,3 | 0,45 | 0,225 | | | 0,3 |
| Caprylyl Glycol | | | | | | | | 0,1 |
| Parfum | 0,25 | 0,3 | 0,4 | 0,5 | 0,55 | 0,42 | 0,5 | 0,4 |
| Sodium Benzoate | 0,35 | 0,2 | 0,5 | 0,25 | | 0,42 | 0,47 | 0,38 |
| Sodium Salicylate | | 0,2 | | | 0,35 | | | |
| Aqua | ad 100 | ad 100 | ad 100 | ad 100 | ad 100 | ad 100 | ad 100 | ad 100 |

| **Inhaltsstoffe** | **Bsp. 14** | **Bsp. 15** | **Bsp. 16** | **Bsp. 17** | **Bsp. 18** | **Bsp. 19** | **Bsp. 20** |
|---|---|---|---|---|---|---|---|
| Sodium Lauroyl Methyl Isethionate | 1,8 | 1,5 | 1,4 | 1,2 | 1,45 | 1,3 | 1,1 |
| Sodium Methyl Cocoyl Taurate | 1 | 1,2 | 1,8 | 1,4 | 1,6 | 1,1 | 1,15 |
| Cocamidopropyl Betaine | 4,8 | 4,4 | 4,6 | 4,1 | 4,42 | 4,4 | 4,3 |
| Decyl Glycoside | | | | 1 | | | |
| Lauryl Glucoside | | | 0,2 | | | 0,5 | |
| Sodium Cocoamphoacetate | | 0,2 | | | | | |
| Polyquaternium-7 | 0,4 | 0,2 | 0,225 | | 0,225 | | |
| Hydroxypropyl Guar Hydroxypropyltrimonium Chloride | | | | | | 0,15 | |
| Guar Hydroxypropyltrimonium Chloride | | | | | | | 0,1 |
| Polyquaternium-10 | | | | 0,1 | | | |
| Parfum | 0,5 | 0,5 | 0,6 | 0,5 | 0,5 | 0,5 | 0,5 |
| Glycerin | | | | | | 2 | 3 |
| Citric Acid | | | | | 0,45 | | |
| Sodium Benzoate | 0,45 | 0,45 | 0,45 | 0,45 | 0,45 | 0,45 | 0,45 |
| Sodium Chloride | 0,25 | 0,5 | | | | | |
| Aqua | ad 100 | ad 100 | ad 100 | ad 100 | ad 100 | ad 100 | ad 100 |

## Patentansprüche

1. Kosmetische Reinigungszubereitung enthaltend, bezogen auf das Gesamtgewicht der Reinigungszubereitung,
a) Sodium Lauroyl Methyl Isethionate,
b) Sodium Methyl Cocoyl Taurate, und
c) Cocamidopropylbetaine,
**dadurch gekennzeichnet, dass** Cocamidopropylbetaine in einem Anteil von 3,5 Gew.- % bis 6 Gew.-% enthalten ist und Wasser in einem Anteil von 60 Gew.-% bis 94 Gew.-% enthalten ist.

2. Reinigungszubereitung nach Anspruch 1 **dadurch gekennzeichnet, dass** der Anteil an Sodium Lauroyl Methyl Isethionate in der kosmetischen Reinigungszubereitung von 0,5 Gew.-% bis 5 Gew.-%, jeweils bezogen auf das Gesamtgewicht der Reinigungszubereitung, beträgt.

3. Reinigungszubereitung nach einem der vorhergehenden Ansprüche **dadurch gekennzeichnet, dass** der Anteil von Sodium Methyl Cocoyl Taurate von 0,5 Gew.-% bis 4 Gew.-%, jeweils bezogen auf das Gesamtgewicht der Reinigungszubereitung, beträgt.

4. Reinigungszubereitung nach einem der vorhergehenden Ansprüche **dadurch gekennzeichnet, dass** die Reinigungszubereitung einen pH-Wert im Bereich von 4,4 bis 6,5 und bevorzugt von 5 bis 5,5 aufweist.

5. Reinigungszubereitung nach einem der vorhergehenden Ansprüche **dadurch gekennzeichnet, dass** die Reinigungszubereitung keine mit Polyethylen Glykol substituierte Substanzen enthält.

6. Reinigungszubereitung nach einem der vorhergehenden Ansprüche **dadurch gekennzeichnet, dass** die Reinigungszubereitung keine Acrylat-basierten Polymere enthält.

7. Reinigungszubereitung nach einem der vorhergehenden Ansprüche **dadurch gekennzeichnet, dass** die Reinigungszubereitung keine zusätzlichen Polymere auf Basis von Polysacchariden und/oder Stärken enthält.

8. Reinigungszubereitung nach einem der vorhergehenden Ansprüche **dadurch gekennzeichnet, dass** Reinigungszubereitung kein Natriumlaurylethersulfat oder Natriumaurylsulfat enthält.

9. Reinigungszubereitung nach einem der vorhergehenden Ansprüche **dadurch gekennzeichnet, dass** diese Natriumbenzoat enthält und der Anteil von Natriumbenzoat bevorzugt von 0,1 Gew.-% bis 0,5 Gew.-% bezogen auf das Gesamtgewicht der Reinigungszubereitung beträgt.

10. Reinigungszubereitung nach einem der vorhergehenden Ansprüche **dadurch gekennzeichnet, dass** Natriumchlorid in einem Anteil von weniger als 0,1 Gew.-% bezogen auf das Gesamtgewicht der Reinigungszubereitung der Reinigungszubereitung zugesetzt wird, wobei es insbesondere bevorzugt ist, wenn der Reinigungszubereitung kein Natriumchlorid zugesetzt wird.

11. Reinigungszubereitung nach einem der vorhergehenden Ansprüche **dadurch gekennzeichnet, dass**, sofern die Reinigungszubereitung eine Ölphase aufweist, der Gesamtanteil der Ölphase bezogen auf das Gesamtgewicht der Reinigungszubereitung weniger als 1 Gew.-%, bevorzugt weniger als 0,8 Gew.-%, weiterhin bevorzugt weniger als 0,7 Gew.-% und insbesondere bevorzugt weniger als 0,6 Gew.-% beträgt.

12. Reinigungszubereitung nach einem der vorhergehenden Ansprüche **dadurch gekennzeichnet, dass** die Reinigungszubereitung zusätzlich Polyquaternium-7 enthält und der Anteil von Polyquaternium-7 von 0,05 Gew.-% bis 0,3 Gew.-% bezogen auf das Gesamtgewicht der kosmetischen Reinigungszubereitung beträgt.

## Claims

1. Cosmetic cleansing preparation comprising, based on the total weight of the cleansing preparation,
a) Sodium Lauroyl Methyl Isethionate,
b) Sodium Methyl Cocoyl Taurate, and
c) cocamidopropyl betaine,
**characterized in that** cocamidopropyl betaine is present in a proportion from 3.5% by weight to 6% by weight and water is present in a proportion from 60% by weight to 94% by weight.

2. Cleansing preparation according Claim 1, **characterized in that** the proportion of Sodium Lauroyl Methyl Isethionate in the cosmetic cleansing preparation is from 0.5% by weight to 5% by weight, in each case based on the total weight of the cleansing preparation.

3. Cleansing preparation according to either of the preceding claims, **characterized in that** the proportion of Sodium Methyl Cocoyl Taurate is from 0.5% by weight to 4% by weight, in each case based on the total weight of the cleansing preparation.

4. Cleansing preparation according to any of the preceding claims, **characterized in that** the cleansing preparation has a pH in the range from 4.4 to 6.5 and preferably from 5 to 5.5.

5. Cleansing preparation according to any of the preceding claims, **characterized in that** the cleansing preparation does not comprise any substances substituted with polyethylene glycol.

6. Cleansing preparation according to any of the preceding claims, **characterized in that** the cleansing preparation does not comprise any acrylate-based polymers.

7. Cleansing preparation according to any of the preceding claims, **characterized in that** the cleansing preparation does not comprise any additional polymers based on polysaccharides and/or starches.

8. Cleansing preparation according to any of the preceding claims, **characterized in that** the cleansing preparation does not comprise any sodium lauryl ether sulfate or sodium lauryl sulfate.

9. Cleansing preparation according to any of the preceding claims, **characterized in that** it comprises sodium benzoate and the proportion of sodium benzoate is preferably from 0.1% by weight to 0.5% by weight based on the total weight of the cleansing preparation.

10. Cleansing preparation according to any of the preceding claims, **characterized in that** sodium chloride is added to the cleansing preparation in a proportion of less than 0.1% by weight based on the total weight of the cleansing preparation, it being especially preferred if no sodium chloride is added to the cleansing preparation.

11. Cleansing preparation according to any of the preceding claims, **characterized in that**, if the cleansing preparation has an oil phase, the total proportion of the oil phase based on the total weight of the cleansing preparation is less than 1% by weight, preferably less than 0.8% by weight, further preferably less than 0.7% by weight and especially preferably less than 0.6% by weight.

12. Cleansing preparation according to any of the preceding claims, **characterized in that** the cleansing preparation additionally comprises Polyquarternium-7 and the proportion of Polyquarternium-7 is from 0.05% by weight to 0.3% by weight based on the total weight of the cosmetic cleansing preparation.

## Revendications

1. Préparation nettoyante cosmétique contenant, par rapport au poids total de la préparation nettoyante,
a) du Sodium Lauroyl Methyl Isethionate
b) du Sodium Methyl Cocoyl Taurate, et
c) de la Cocamidopropylbetaine, **caractérisée en ce que** la Cocamidopropylbetaine est contenue en une quantité de 3,5 % en poids à 6 % en poids et de l'eau est contenue en une quantité de 60 % en poids à 94 % en poids,

2. Préparation nettoyante selon la revendication 1, **caractérisée en ce que** la quantité de Sodium Lauroyl Méthyl Isethionate représente, dans la préparation nettoyante cosmétique, 0,5 % en poids à 5 % en poids, respectivement par rapport au poids total de la préparation nettoyante.

3. Préparation nettoyante selon l'une quelconque des revendications précédentes, **caractérisée en ce que** la quantité de Sodium Methyl Cocoyl Taurate représente, dans la préparation nettoyante cosmétique, 0,5 % en poids à 4 % en poids, respectivement par rapport au poids total de la préparation nettoyante.

4. Préparation nettoyante selon l'une quelconque des revendications précédentes, **caractérisée en ce que** la préparation nettoyante présente un pH dans la plage de 4,4 à 6,5 et préférablement de 5 à 5,5.

5. Préparation nettoyante selon l'une quelconque des revendications précédentes, **caractérisée en ce que** la préparation nettoyante ne contient pas de substances substituées à base de polyéthylène glycol.

6. Préparation nettoyante selon l'une quelconque des revendications précédentes, **caractérisée en ce que** la préparation nettoyante ne contient pas de polymères à base d'acrylate.

7. Préparation nettoyante selon l'une quelconque des revendications précédentes, **caractérisée en ce que** la préparation nettoyante ne contient pas de polymères supplémentaires à base de polysaccharides et/ou d'amidons.

8. Préparation nettoyante selon l'une quelconque des revendications précédentes, **caractérisée en ce que** la préparation nettoyante ne contient pas de lauryl éther sulfate de sodium ni de laurylsulfate de sodium.

9. Préparation nettoyante selon l'une quelconque des revendications précédentes, **caractérisée en ce qu'**elle contient du benzoate de sodium et la quantité de benzoate de sodium est préférablement de 0,1 % en poids à 0,5 % en poids par rapport au poids total de la préparation nettoyante.

10. Préparation nettoyante selon l'une quelconque des revendications précédentes, **caractérisée en ce que** du chlorure de sodium est ajouté à la préparation nettoyante en une quantité inférieure à 0,1 % en poids par rapport au poids total de la préparation nettoyante, sachant qu'il est particulièrement préférable qu'aucun chlorure de sodium ne soit ajouté à la préparation nettoyante.

11. Préparation nettoyante selon l'une quelconque des revendications précédentes, **caractérisée en ce que**, dans la mesure où la préparation nettoyante présente une phase huileuse, la quantité totale de la phase huileuse par rapport au poids total de la préparation nettoyante est inférieure à 1 % en poids, préférablement inférieure à 0,8 % en poids, plus préférablement inférieure à 0,7 % en poids et particulièrement préférablement inférieure à 0,6 % en poids.

12. Préparation nettoyante selon l'une quelconque des revendications précédentes, **caractérisée en ce que** la préparation nettoyante contient du polyquaternium-7 et **en ce que** la quantité de polyquaternium-7 est préférablement de 0,05 % en poids à 0,3 % en poids par rapport au poids total de la préparation nettoyante cosmétique.
